(19) Europäisches Patentamt / European Patent Office / Office européen des brevets

(11) **EP 3 273 775 B1**

(12) **EUROPEAN PATENT SPECIFICATION**

(45) Date of publication and mention of the grant of the patent:
**13.11.2019 Bulletin 2019/46**

(51) Int Cl.:
*A01K 67/00* (2006.01)    *C12Q 1/02* (2006.01)
*G01N 33/569* (2006.01)

(21) Application number: **16712303.3**

(22) Date of filing: **22.03.2016**

(86) International application number:
**PCT/EP2016/056196**

(87) International publication number:
**WO 2016/150931 (29.09.2016 Gazette 2016/39)**

(54) **PROCESS TO INFECT CRUSTACEANS WITH INFECTIOUS AGENTS**

VERFAHREN ZUM INFIZIEREN VON KRUSTENTIEREN MIT INFEKTIONSERREGERN

PROCÉDÉ PERMETTANT D'INFECTER DES CRUSTACÉS AVEC DES AGENTS INFECTIEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.03.2015 EP 15160546**

(43) Date of publication of application:
**31.01.2018 Bulletin 2018/05**

(73) Proprietor: **Universiteit Gent**
**9000 Gent (BE)**

(72) Inventor: **NAUWYNCK, Hans**
**9930 Zomergem (BE)**

(56) References cited:
• SAULNIER D ET AL: "Experimental infection models for shrimp vibriosis studies: a review", AQUACULTURE, ELSEVIER, AMSTERDAM, NL, vol. 191, no. 1-3, 20 November 2000 (2000-11-20), pages 133-144, XP027252411, ISSN: 0044-8486 [retrieved on 2000-11-20]
• RAHMAN M M ET AL: "Effect of high water temperature (33 <o>C) on the clinical and virological outcome of experimental infections with white spot syndrome virus (WSSV) in specific pathogen-free (SPF) Litopenaeus vannamei", AQUACULTURE, ELSEVIER, AMSTERDAM, NL, vol. 261, no. 3, 1 December 2006 (2006-12-01), pages 842-849, XP027903625, ISSN: 0044-8486 [retrieved on 2006-12-01]

• MACEY B M ET AL: "Clearance of Vibrio campbellii injected into the hemolymph of Callinectes sapidus, the Atlantic blue crab: The effects of prior exposure to bacteria and environmental hypoxia", FISH AND SHELLFISH IMMUNOLOGY, ACADEMIC PRESS, LONDON, GB, vol. 25, no. 6, 1 December 2008 (2008-12-01), pages 718-730, XP025672825, ISSN: 1050-4648, DOI: 10.1016/J.FSI.2008.02.009 [retrieved on 2008-02-19]
• GROSS ET AL: "Glucose absorption from the urinary bladder of a crab", COMPARATIVE BIOCHEMISTRY AND PHYSIOLOGY, PERGAMON, vol. 20, no. 1, 1 January 1967 (1967-01-01), pages 313-317, XP023565330, ISSN: 0010-406X, DOI: 10.1016/0010-406X(67)90746-3 [retrieved on 1967-01-01]
• KAMEMOTO F ET AL: "Cholinesterase activities and sodium movement in the crayfish kidney", COMPARATIVE BIOCHEMISTRY AND PHYSIOLOGY, PERGAMON, vol. 7, no. 1-2, 1 September 1962 (1962-09-01), pages 81-87, XP025201547, ISSN: 0010-406X, DOI: 10.1016/0010-406X(62)90030-0 [retrieved on 1962-09-01]
• STERN S ET AL: "Osmotic and ionic regulation of the prawn Macrobrachium rosenbergii (De Man) adapted to varying salinities and ion concentrations", COMPARATIVE BIOCHEMISTRY AND PHYSIOLOGY. PART A. COMPARATIVE PHYSIOLOGY, ELSEVIER SCIENCE LTD, US, vol. 86, no. 2, 1 January 1987 (1987-01-01), pages 373-379, XP025403249, ISSN: 0300-9629, DOI: 10.1016/0300-9629(87)90345-8 [retrieved on 1987-01-01]

- **AWANTHA DISSANAYAKE ET AL: "Seasonal differences in the physiology of(Crustacea: Decapoda) from estuaries with varying levels of anthropogenic contamination", ESTUARINE, COASTAL AND SHELF SCIENCE, NEW YORK, NY, US, vol. 93, no. 4, 25 April 2011 (2011-04-25) , pages 320-327, XP028238177, ISSN: 0272-7714, DOI: 10.1016/J.ECSS.2011.04.014 [retrieved on 2011-05-11]**

- **FREIRE C A ET AL: "A structure-function analysis of ion transport in crustacean gills and excretory organs", COMPARATIVE BIOCHEMISTRY AND PHYSIOLOGY. PART A, MOLECULAR AND INTEGRATIVE PHYSIOLOGY, ELSEVIER SCIENCE, NEW YORK, NY, US, vol. 151, no. 3, 1 November 2008 (2008-11-01), pages 272-304, XP025474713, ISSN: 1095-6433, DOI: 10.1016/J.CBPA.2007.05.008 [retrieved on 2008-10-01]**

**Description**

Technical field of invention

The present invention relates to the field of aquaculture of commercially important crustaceans such as penaeid shrimp. The present invention discloses a process to infect crustaceans via inoculating pathogens directly into the external pore of the antennal gland of said crustaceans. This new portal of entry for pathogens can be used to investigate and/or prevent infection of crustaceans with pathogens such as white spot syndrome virus.

Background art

During the last decades, aquaculture of crustaceans such as penaeid shrimp has expanded in many (sub)tropical countries around the world. Since 2010, the yearly production volume of for example penaeid shrimp has passed 3 million metric tons with a value of more than 12 billion dollars. However, the intensification of the industry is accompanied by serious infectious diseases, such as white spot syndrome and Vibrio infections. Up to date, despite many attempts by both academic research institutes and commercial companies, no effective control measures have been developed to control diseases in crustacean farms. One of the key problems behind the lack of control measures, is the fragmentary knowledge on the factors determining the susceptibility of the host to infection. For example, very little is understood how pathogens manage to enter the host.

With regard to infection with white spot syndrome virus for example, it has become generally accepted that viral transmission between shrimp can occur via 3 routes: 1) by oral consumption of infected tissues, 2) via virus-containing water, and possibly 3) vertically from broodstock to offspring.

Several experimental studies (reviewed by Corteel, 2013) demonstrated that feeding of infected shrimp tissues resulted in infection. However, many authors needed to administer infected tissues in several feedings, which raises the question whether oral consumption is a major transmission route under all natural circumstances. Other authors further concluded -under experimental conditions-that ingestion of infected tissues is more effective in transmitting the virus between shrimp than immersion in infected water indicating that also contact with infected water is unlikely to be a major transmission route in nature. Furthermore, it should be noted that all the tissues known to be susceptible to infection with for example white spot syndrome virus (i.e. tissues of ectodermal or mesodermal origin) are protected from the outside world by an impenetrable cuticula.

The antennal gland, which is still poorly described in crustaceans such as penaeid shrimp, is the main excretory system organ of these animals. The latter system is composed of several parts: a bladder, the labyrinth, the coelomosac, the nephridial canal and the nephropore. Similar as for kidneys, the main functions of the antennal gland are osmo-regulation, acid/base homeostasis and detoxification. Close to the base of the antennae, the bladder expels urine or nitrogenous waste through an external pore, also called the nephropore (reviewed by Corteel 2013).

Lin et al. (2000) described that urine production in P. monodon dramatically increased after the animals were transferred in seawater having a lower salinity.

In shrimp aquaculture industry, farmers often observe an outbreak of white spot syndrome after heavy rain and a corresponding drop in seawater salinity.

Description of invention

The present invention relates to the surprising finding that Crustaceans can be infected with a pathogen via inoculating said pathogen directly into the external pore of the antennal gland of said crustacean. The latter pore has been solely described to expel urine and the antennal glands connected to said pore are described to control turgor, osmolality and water-ion content within the crustaceans body. The present invention discloses -as non-limiting and proof-of-concept examples-that both White Spot Syndrome Virus (WSSV) and *Vibrio campbellii,* two major pathogens in shrimp, are easily infecting shrimp upon so-called 'intra-antennal-gland-bladder' inoculation (= inoculation into the external pore of the antennal gland). The present invention further demonstrates that WSSV can infect shrimp much more easilyvia an intra-antennal-gland-bladder way than via oral way or via immersion. For the oral route, $10^{7.3-7.7}$ infectious virus is necessary for infection when virus suspension is directly intubated in the alimentary tract and even $10^{8.0-8.2}$ infectious virus is necessary when given via feed. In contrast, $10^{1.5-2.1}$ infectious virus is sufficient when virus is directly inoculated in the external pore of the antennal gland. It is hereby important to note that the latter concentration range is comparable to concentrations that are present under natural conditions, which indicates that the external pores of said antennal glands might be the natural ports of entry for pathogens.

Therefore the present invention relates to a process to infect a crustacean with an infectious agent comprising:

- collecting said crustacean,

- collecting said infectious agent,

- removing urine via the external pore (nephropore) of the antennal gland of said crustacean, and

- inoculating an amount of said infectious agent into the external pore of the antennal gland of said crustacean.

The term 'Crustacean' means an animal belonging to the group (or phylum) of arthropods, and specifically includes such familiar animals as shrimp, prawns, crayfish and crabs. Crustaceans have an exoskeleton, which they molt to grow. They are distinguished from other groups of arthropods, such as insects, myriapods and chelicerates, by the possession of biramous (two-parted) limbs, and by the nauplius form of their larvae.

The present invention specifically relates to a process as described above wherein said crustacean is a shrimp, a prawn or a crab. More specifically, the present invention relates to a process as described above wherein said shrimp is a Penaeid shrimp or wherein said prawn is a Palaemonid prawn (which is sometimes also denominated as a Paleamonid shrimp). Even more specifically, the present invention relates to a process as described above wherein said Penaeid shrimp is (Lito)penaeus vannamei, Penaeus monodon, Penaeus indicus or Penaeus chinensis, or wherein said Palaemonid prawn or shrimp is a Macrobrachium species, or, wherein said crab is a Chinese mitten crab (Eriocheir sinensis) or mud crab (Scylla serrata).

The term 'infectious agent' means a microorganism such as a virus, bacterium, prion, phage or fungus, that causes disease in crustaceans. Said infectious agent can be isolated from an infected crustacean and subsequently used to produce a stock of infectious agent as described in Jiravanichpaisal, Bangyeekhun et al. (2001). The term 'infectious agent' particularly relates to the most prevalent infectious agents in crustaceans such as white spot syndrome virus and Vibrio's causing the so-called 'early mortality syndrome'. Non-limiting examples of said Vibrio's are *Vibrio parahaemolyticus, Vibrio harveyii, Vibrio anguilarum* and *Vibrio campbellii.*

The term 'a virus' means a small infectious agent that replicates only inside the living cells of other organisms. Viruses can infect all types of life forms, from animals and plants to bacteria and archaea. Examples are White Spot Syndrome Virus, Taura virus and Gill associated virus.

Hence, the present invention specifically relates to a process as describe above, wherein said virus is white spot syndrome virus.

The invention further specifically relates to a process as described above, wherein said infectious agent is a bacterium.

The term 'a bacterium' means a member of the kingdom of prokaryotic microorganisms. Typically a bacterium is a few micrometres in length. Bacteria have a wide range of shapes, ranging from spheres to rods and spirals. Prokaryotic life further consists of two separate domains, originally called *Eubacteria* and *Archaebacteria,* but now called *Bacteria* and *Archaea.*

The present invention further more specifically relates to a process as describe above, wherein said bacterium is a bacterium belonging to the genus Vibrio. 'Vibrio' is a genus of Gram-negative bacteria possessing a curved rod shape. Several species of the genus Vibrio can cause foodborne infection, usually associated with eating undercooked seafood. Vibrio's are typically found in saltwater, are facultative anaerobes that test positive for oxidase and do not form spores. All members of the genus are motile and have polar flagella with sheaths. As indicated above, the present invention relates in particular to Vibrio's causing the so-called 'early mortality syndrome'.

The term 'a process to infect Crustacean' means a process in which the infectious agent is transferred to or inoculated into a host, the host being said crustacean. The host is successfully infected when the infectious agent starts to multiply in its host. Verification of infection can be done by assessing mortality of said crustacean, or, by sampling tissue of said crustacean and demonstrating the presence of infectious agent by indirect immunofluorescence (IIF) as is for example described by Escobedo-Bonilla et al. (2005), or, by any other way known to a skilled person.

The term 'collecting said crustacean' means sampling a crustacean and putting it into a position so that it can be easily infected by an infectious agent. This can for example be undertaken by physically isolating the crustacean in small groups or as individuals and subsequently positioning them so that the pore at the base of the antennal gland can be easily accessed.

The term 'collecting said infectious agent' means taking an amount of infectious agent from the above mentioned stock of infectious agent and diluting this stock to a titer at which a certain percentage of the exposed crustaceans become infected. Determination of the 'virus infection titers' ($SID_{50}$/ml) can be calculated based on the method of Reed and Muench (1938). Escobedo-Bonilla (2005) describes in detail how the median infection titer of WSSV, as an example of an infectious agent, can be determined. One shrimp infectious dose with 50% endpoint (SID50) is the amount of infectious virus that causes an infection in 50% of the exposed crustaceans using a certain inoculation route.

The terms "the external pore of the antennal gland of said Crustacean" means the external pore of the antennal gland, also called the nephropore located close to the base of the antenna, which is used to expel urine or nitrogenous waste from the bladder (Felghauer 1992 and Corteel 2013).

The present invention further specifically relates to a process as described above wherein said inoculation is undertaken with a catheter or a needle attached to a syringe containing said amount of infectious agent.

More specifically, the present invention relates to a process as described above wherein said syringe is a 0.1 ml syringe attached to a 24G catheter.

The term 'catheter' means any device that resembles a thin tube and that can be used to transfer an amount of infectious agent into the antennal gland of a crustacean via the external pore of the said antennal gland. A typical 'catheter' is a suitable thin plastic tube connected to a syringe containing an amount of infectious agent. An -non-limiting- example of the latter is a 0.1 ml syringe attached to a 0.64 mm x 19 mm catheter (terumo surflo w-24G) catheter.

The term 'removing urine' means that urine is removed from the antennal gland/bladder via the nephropore. The latter can be undertaken by gently introducing the tip of a catheter in the nephropore for a few seconds. For example, the tip of a 0.64 mm x 19 mm catheter (terumo surflo w-24G) can be introduced into the nephropore (about 1mm). The catheter can then be kept stable for few seconds until the urine fills the catheter.

The present invention further relates to a process to screen for crustaceans which are resistant to an infectious agent comprising infecting said crustaceans using any of the processes as described above and determining resistance of said crustaceans to said infection.

Indeed, the methods to infect crustaceans as described above can be used to investigate and/or determine which species or variety of crustacean is significantly more or less resistant to a particular pathogen or infectious agent as described above when compared to a related species or variety of crustacean. As such the methods as described in the present invention can be used to screen or select for resistant crustaceans.

With the term 'resistance' is meant any natural- or immune-mediated resistance from the host/crustacean towards an infectious agent so that an infectious agent is not- or less capable to infect, colonize and/or multiply within said host. 'Resistance' can be determined by the mortality rate or rate of infection as described above.

Moreover, the present invention relates to a process to screen for compounds modulating resistance of crustaceans to an infectious agent comprising exposing said crustaceans to said compounds, infecting said crustaceans using a process as described above and determining resistance of said crustaceans to infection compared to resistance of crustaceans which are not exposed to said compounds.

Indeed, the methods to infect crustaceans as described above can be used to investigate and/or determine if compounds such as particular molecules present in feed, particular drugs, particular probiotics or certain water pollutants -or a mixture of said compounds- can modulate (= significantly increase or decrease) resistance of a crustacean to pathogens or to a particular selection of pathogens. The latter usage may comprise:

- exposing said crustaceans to a range of concentrations of said compounds for a specific period of time,

- infecting said crustaceans using a process as described above, and

- determining resistance -as defined above- of said crustaceans to infection compared to resistance of control crustaceans which are not exposed to said compounds.

The present invention will hereby following be illustrated by non-limiting examples.

Examples

**Example 1**

**Comparison of White Spot Syndrome Virus (WSSV) infectivity in shrimp inoculated via intramuscular route, via peroral route using intubation or feeding**

At present, several routes how shrimp become infected with WSSV have been described. Inoculating the virus via intramuscular inoculation is very efficient in infecting shrimp, leading to high mortality. Once the virus starts its replication, the animal seems not to have an immunological reaction to control infection. Infecting animals via immersion in WSSV-contaminated water is not very efficient and is mainly possible at molting. Infecting animals via intubation and via feeding has been reported, but the efficiency with which the animals can become infected has not been assessed. Therefore, we firstly examined the infectivity of WSSV upon inoculation via intramuscular route, via peroral intubation and via feeding.

**Material and Methods**

**1. WSSV preparation**

**1.1. Preparation of a starting WSSV stock**

WSSV Thai-1 used in this study was collected from infected *Penaeus monodon* in Thailand in 1996 and amplified in crayfish *Pacifastacus leniusculus* (Jiravanichpaisal et al., 2001). A homogenate of WSSV infected crayfish gills kindly donated by P. Jiravanichpaisal and K. Soderhall (Uppsala University, Sweden) was inoculated in SPF *P.vannamei* juveniles to produce virus stock. The median infectious titer of the stock was $10^{6.6}$ shrimp infectious dose 50% end point $(SID_{50})$ ml$^{-1}$ as determined by *in vivo* intramuscular titration (Escobedo-Bonilla et al., 2005).

**1. 2. Preparation of different WSSV stocks**

The starting WSSV stock (prepared in 1.1) was diluted $10^{-2}$ in phosphate-buffered saline (PBS) pH 7.4 and was intramuscularly injected into SPF *P. vannamei* juveniles to amplify the virus. Then, moribund shrimp were collected and confirmed to be WSSV positive by indirect immunoflourescence (IIF). Shell, hepatopancreas and gut were removed and the remaining bodies were cut longitudinally into two parts. The left body parts were homogenized at 5000 rpm for 5 minutes using ultra-turrax IKA T 25. Then, the homogenate was further minced by serial passages through needles with a decreasing diameter (1.2, 0.9 and 0.55 x 20 mm), aliquoted and stored at -70 °C (WSSV stock 1). The right body parts were cut into very small pieces (squares of 0.5-1mm), mixed and stored at -70 °C (WSSV stock 2).

**2. Challenge tests for the comparison of the infectivity of WSSV in shrimp upon inoculation via intramuscular route, via peroral intubation or via feeding**

The aim of this first experiment was to compare the infectivity of WSSV in shrimp upon inoculation via intramuscular route, via peroral intubation and via natural feeding. Shrimp in inter-molt with a mean body weight (MBW) of 4.86 ± 0.37 g were acclimated individually in a 10 liter-tank and divided in three groups. Shrimp were starved for 24h prior to inoculation. Twenty shrimp in the first group were intramuscularly injected with 50mg of a 10-fold serial dilution ($10^{-6}$, $10^{-7}$, $10^{-8}$, $10^{-9}$; 5 shrimp per dilution) of WSSV stock 1. Twenty shrimp in the second group were perorally inoculated with 50mg of a 10-fold serial dilution ($10^{0}$, $10^{-1}$, $10^{-2}$, $10^{-3}$; 5 shrimp per dilution) of WSSV stock 1 using a 0,74 x 19 mm - 24G Surflor-W catheter (the 10-fold serial dilution was prepared by mixing 1 part of WSSV stock with 9 parts of pathogen-free shrimp minced tissue). Thirty shrimp in the third group were individually fed with 0.5, 5, 50, 100, 250 or 500 mg of WSSV stock 2; 5 shrimp dose). Shrimp were given 1 meal for the doses of 0.5, 5, 50 and 100 mg, 3 meals for the dose of 250 mg and 5 meals for the dose of 500 mg. The time interval between two meals was 1h. After inoculation, shrimp were housed individually and observed for 5 days. Dead and moribund shrimp were collected every 12h and the experiment was terminated at 120 hpi. Moribund, dead and euthanized surviving shrimp were processed to control the WSSV infection status by IIF.

The experiment was repeated once.

**Results (Table 1)**

*Intramuscular inoculation route*

Experiment 1 - Among the groups of shrimp injected with WSSV stock 1, diluted $10^{-6}$ to $10^{-9}$, 5 out of 5 shrimp in the $10^{-6}$ dilution group, 4 out of 5 shrimp in the $10^{-7}$ group and 1 out of 5 shrimp in the $10^{-8}$ group died between 36 and 84 hpi. All the other shrimp in the $10^{-7}$, $10^{-8}$ and $10^{-9}$ groups survived until the end of the experiment.

Experiment 2 - In the second experiment, the number of dead animals in the different dilution groups was the same.

*Inoculation via peroral intubation*

Experiment 1 - Of shrimp orally inoculated with WSSV stock 1, diluted 10° to $10^{-3}$, 2 out of 5 shrimp in the dilution of 10° died. The other shrimp in the 10° group and all shrimp in the $10^{-1}$, $10^{-2}$ and $10^{-3}$ groups survived until the end of the experiment.

Experiment 2 - 3 out of 5 shrimp in the 10° group died between 48 and 72 hpi. All other shrimp survived until the end of the experiment.

*Inoculation via feeding*

Experiment 1 - Among the groups of 5 shrimp, fed 0.5, 5, 50, 100, 250 or 500mg of WSSV infected tissue, 0, 1, 0, 1, 2 and 3 out of 5 shrimp died, respectively.

Experiment 2 - In this experiment, 0, 0, 1, 2, 3 and 4 deaths out of 5 shrimp were recorded, in the 0.5, 5, 50, 100, 250 or 500mg groups, respectively. The other shrimp survived until the end of the experiment.

In all experiments, IIF analysis revealed that all dead animals were WSSV positive while the other shrimp that survived until the end of the experiment at 120hpi were WSSV negative.

The virus infectivity titers determined upon intramuscular injection, peroral intubation and via feeding were $10^{8.8}$, $10^{1.1}$ and $10^{0.6}$ $SID_{50}$ $g^{-1}$, respectively, for the first replicate and $10^{8.8}$, $10^{1.5}$ and $10^{0.8}$ $SID_{50}$ $g^{-1}$ for the second replicate.

Table 1. Mortality in shrimp inoculated via intramuscular injection, peroral intubation and via feeding and infection status of dead, moribund and euthanized animals. Virus titers were calculated using the method of Reed and Muench.

| Experiment N° | Inoculation route | Dilution of WSSV | No. of shrimp | No. of mortality at different time points (hpi) | | | | | | | | | Infection status by IIF | Virus titer $SID_{50}$/g |
|---|---|---|---|---|---|---|---|---|---|---|---|---|---|---|
| | | | | 24 | 36 | 48 | 60 | 72 | 84 | 96 | 120 | Total | | |
| 1 | Intra-muscular | $10^{-6}$ | 5 | | 2 | 2 | 1 | | | | | 5 | 5 | $10^{8.8}$ |
| | | $10^{-7}$ | 5 | | 1 | 1 | 1 | | 1 | | | 4 | 4 | |
| | | $10^{-8}$ | 5 | | | | 1 | | | | | 1 | 1 | |
| | | $10^{-9}$ | 5 | | | | | | | | | 0 | 0 | |
| | Peroral intubation | $10^{0}$ | 5 | | | 1 | 1 | | | | | 2/5 | 2/5 | $10^{1.1}$ |
| | | $10^{-1}$ | 5 | | | | | | | | | 0/5 | 0/5 | |
| | | $10^{-2}$ | 5 | | | | | | | | | 0/5 | 0/5 | |
| | | $10^{-3}$ | 5 | | | | | | | | | 0/5 | 0/5 | |
| | Feeding | 0.5 | 5 | | | | | | | | | 0/5 | 0/5 | $10^{0.6}$ |
| | | 5 | 5 | | 1 | | | | | | | 1/5 | 1/5 | |
| | | 50 | 5 | | | | | | | | | 0/5 | 0/5 | |
| | | 100 | 5 | | 1 | | | | | | | 1/5 | 1/5 | |
| | | 250 | 5 | | 1 | 1 | | | | | | 2/5 | 2/5 | |
| | | 500 | 5 | | | | | 3 | | | | 3/5 | 3/5 | |

(continued)

| Experiment N° | Inoculation route | Dilution of WSSV | No. of shrimp | No. of mortality at different time points (hpi) | | | | | | | | | Infection status by IIF | Virus titer $SID_{50}/g$ |
|---|---|---|---|---|---|---|---|---|---|---|---|---|---|---|
| | | | | 24 | 36 | 48 | 60 | 72 | 84 | 96 | 120 | Total | | |
| 2 | Intra-muscular | $10^{-6}$ | 5 | | 1 | 3 | | 1 | | | | 5/5 | 5/5 | $10^{8.8}$ |
| | | $10^{-7}$ | 5 | | | 1 | 2 | 1 | | | | 4/5 | 4/5 | |
| | | $10^{-8}$ | 5 | | | | 1 | | | | | 1/5 | 1/5 | |
| | | $10^{--9}$ | 5 | | | | | | | | | 0/5 | 0/5 | |
| | Peroral intubation | $10^{0}$ | 5 | | | | 2 | 1 | | | | 3/5 | 3/5 | $10^{1.5}$ |
| | | $10^{-1}$ | 5 | | | | | | | | | 0/5 | 0/5 | |
| | | $10^{-2}$ | 5 | | | | | | | | | 0/5 | 0/5 | |
| | | $10^{-3}$ | 5 | | | | | | | | | 0/5 | 0/5 | |
| | Feeding | 0.5 | 5 | | | | | | | | | 0/5 | 0/5 | $10^{0.8}$ |
| | | 5 | 5 | | | | | | | | | 0/5 | 0/5 | |
| | | 50 | 5 | | | | 1 | | | | | 1/5 | 1/5 | |
| | | 100 | 5 | | | | 2 | | | | | 2/5 | 2/5 | |
| | | 250 | 5 | | 2 | 1 | | | | | | 3/5 | 3/5 | |
| | | 500 | 5 | | 1 | | 2 | 1 | | | | 4/5 | 4/5 | |

This experiment demonstrates that shrimp can be infected via oral route but that this is totally not efficient. $10^{7.3-7.7}$ infectious virus is necessary to get an infection via intubation and even $10^{8.0-8.2}$ infectious virus is necessary to get an infection via oral route using contaminated feed.

**Example 2: The antennal gland** is **the main entry port for White Spot Syndrome Virus in shrimp Susceptibility of shrimp to WSSV infection upon antennal gland inoculation**

WSSV has a lot of problems to infect shrimp via peroral route. In the past, it was also shown that it is also extremely difficult to infect shrimp via immersion. These findings are in strong contrast with the situation in the field -when WSSV outbreaks occur- as only a fast and efficient transmission can explain these natural, explosive outbreaks. The present invention relates to finding a natural port of entry. Example 2 looks at the susceptibility of shrimp to WSSV infection via antennal gland inoculation and compares it with intramuscular injection.

**Material and Methods**

Thirty-five shrimp (20.4 $\pm$ 3.3 g) in inter-molt were divided into 2 groups. Shrimp in the first group were injected intramuscularly with 5$\mu$l of a10-fold serial dilution ($10^{-5}$ to $10^{-7}$) of a WSSV stock (Thai-1; $10^{8.6}$SID$_{50}$ ml$^{-1}$). Shrimp in the second group were inoculated with 5$\mu$m of a 10-fold of serial dilution ($10^{-3}$ to $10^{-6}$) of the same WSSV stock into the bladder of the antennal gland, with 5 shrimp per dilution. The intra-antennal-gland-bladder inoculation has been standardized. In brief, shrimp were wrapped in tissue paper and put ventral side up under a stereomicroscope. First, urine was removed from the bladder after gently introducing the tip (1mm) of a 0.64 mm x19 mm catheter (terumo surflo w-24G) in the nephropore. The catheter was kept stable for few seconds until the urine fully filled the catheter. Afterwards, the catheter was removed and replaced with a new 0.64 mm x19 mm catheter (terumo surflo w-24G) connected with a 1ml syringe, filled with virus. Five microliter of virus was injected by gently pressure on the plunger of the syringe. After inoculation, shrimp were placed individually in 10-liter tanks, filled with artificial seawater at a salinity of 35 g l$^{-1}$, with constant aeration and at a temperature of 27$\pm$1°C. Shrimp were fed twice daily with commercial pelleted feed at 3% of body weight. Moribund and dead shrimp were collected every 12 h and the experiment was terminated at 120 hpi. Shrimp samples were processed for detection of WSSV infection using IIF. The experiment was done in triplicate. Virus infectivity titers of the WSSV stock in *P. vannamei* was assessed in shrimp by intramuscular injection or intra-antennal-gland-bladder inoculation.

**Results (Table 2)**

In the three experiments, the virus titers (SID50/ml) upon intramuscular inoculation were: $10^{8.67}$, $10^{8.67}$ and $10^{8.8}$ SID$_{50}$/ml and upon intra-antennal-gland-bladder inoculation: $10^{6.97}$, $10^{6.60}$ and $10^{7.30}$ SID$_{50}$/ml. This means that for infecting a shrimp in the three experiments one needs only $10^{1.70}$, $10^{2.07}$ and $10^{1.50}$ infectious virus, respectively, to infect a shrimp via the intra-antennal-bladder route.

Table 2:

| Experiment No. | Inoculation route | Dilution of WSSV | No. of shrimp | Number of dead animals at ... hpi | | | | | | | | | Infection status-IIF | Virus titer $SID_{50}$ $ml^{-1}$ |
|---|---|---|---|---|---|---|---|---|---|---|---|---|---|---|
| | | | | 24 | 36 | 48 | 60 | 72 | 84 | 96 | 120 | Total | | |
| 1 | Intra-muscular | $10^{-5}$ | 5 | | | 3 | 1 | 1 | | | | 5/5 | 5/5 | $10^{8.67}$ |
| | | $10^{-6}$ | 5 | | | 2 | 2 | | | | | 4/5 | 4/5 | |
| | | $10^{-7}$ | 5 | | | | | | | | | 0/5 | 0/5 | |
| | Intra-antennal-gland-bladder | $10^{-3}$ | 5 | | | 2 | 2 | 1 | | | | 5/5 | 5/5 | $10^{6.97}$ |
| | | $10^{-4}$ | 5 | | | 1 | | 2 | 1 | | | 4/5 | 4/5 | |
| | | $10^{-5}$ | 5 | | | | 1 | 1 | | | | 2/5 | 2/5 | |
| | | $10^{-6}$ | 5 | | | | | | | | | 0/5 | 0/5 | |
| 2 | Intra-muscular | $10^{-5}$ | 5 | | | 3 | 2 | | | | | 5/5 | 5/5 | $10^{8.67}$ |
| | | $10^{-6}$ | 5 | | | 1 | 2 | 1 | | | | 4/5 | 4/5 | |
| | | $10^{-7}$ | 5 | | | | | | | | | 0/5 | 0/5 | |
| | Intra-antennal-gland-bladder | $10^{-3}$ | 5 | | | 3 | 2 | | | | | 5/5 | 5/5 | $10^{6.60}$ |
| | | $10^{-4}$ | 5 | | | 1 | 1 | 1 | | | | 3/5 | 3/5 | |
| | | $10^{-5}$ | 5 | | | | 1 | | | | | 1/5 | 1/5 | |
| | | $10^{-6}$ | 5 | | | | | | | | | 0/5 | 0/5 | |
| 3 | Intra-muscular | $10^{-5}$ | 5 | | 3 | 1 | 1 | | | | | 5/5 | 5/5 | $10^{8.80}$ |
| | | $10^{-6}$ | 5 | | 2 | 1 | 1 | | 1 | | | 5/5 | 5/5 | |
| | | $10^{-7}$ | 5 | | | | | | | | | 0/5 | 0/5 | |
| | Intra-antennal-gland-bladder | $10^{-3}$ | 5 | | | 2 | 2 | 1 | | | | 5/5 | 5/5 | $10^{7.30}$ |
| | | $10^{-4}$ | 5 | | 2 | | 1 | 1 | | | | 4/5 | 4/5 | |
| | | $10^{-5}$ | 5 | | | | 2 | | 1 | | | 3/5 | 3/5 | |
| | | $10^{-6}$ | 5 | | | | | | | | | 0/5 | 0/5 | |

Examples 1 and 2 clearly demonstrate that the antennal gland is a major portal of entry of WSSV in shrimp; the intestinal tract is a minor portal of entry.

**Example 3: The antennal gland is a main entry port for Vibrio in shrimp**

In Example 2 it was shown that White Spot Syndrome Virus can easily infect shrimp via the antennal gland. Example 3 evaluates if *Vibrio campbellii* (LMG21363), an important shrimp bacterial pathogen, can also use this portal of entry.

**Material and Methods**

**Bacteria**

Rifampicin-resistant bacterial strains *Vibrio campbellii* (LMG21363) was obtained from the Laboratory of ARC. From the bacterial stock, 20 $\mu$l was aseptically inoculated to 20 ml MB (concentration of rifampicin 100 mg/L). It was incubated for 12 hours at 27°C in a shaker at 90 rotations per minute (rpm). After 12 hours, 20 $\mu$l of the *Vibrio campbellii* (LMG21363) was subcultured by inoculating it into 20 ml of fresh MB (concentration of rifampicin 100 mg/L) and incubated for another 14 hours under the same conditions. After 14 hours, 10ml bacteria culture was collected and transferred to 15 ml tubes and centrifuged at 2000xg for 10 minutes. Then, the supernatant was discarded and the pellet was resuspended twice with 10 ml of FASW and centrifuged at 2000xg for 10 minutes and the pellet was finally resuspended in 500 $\mu$l FASW and vortexed until homogenized well. The suspension was diluted 100 times and the optical density was determined through spectrophotometer at an absorbance of 600 nm. Based on the following formula the concentration of bacteria was calculated:

$$CFU/ml = (10 \times OD600\text{-}1) \times 10^8$$

A bacterial stock was made with an estimated titer of $10^{10}$ cfu/ml

**Animal inoculations**

Serial dilutions ($10^{-1}$, $10^{-2}$, $10^{-3}$ and $10^{-4}$) of the *Vibrio campbellii* (LMG21363) stock were made in FASW. Then, these dilutions were used to determine the lethal titer with 50% endpoint ($LD_{50}$ ml$^{-1}$) in *P. vannamei* by intramuscular, peroral and antennal gland inoculation. For intramuscular injection, each dilution ($10^{-2}$, $10^{-3}$ and $10^{-4}$) was injected intramuscularly in each of 5 shrimp in a volume of 100 $\mu$l. For the per os inoculation, each dilution ($10^0$, $10^{-1}$ and $10^{-2}$) was intubated in each of 5 shrimp in an inoculation volume of 100 $\mu$l per animal. For the antennal gland inoculation, each dilution ($10^0$, $10^{-1}$ and $10^{-2}$) was inoculated in each of 5 shrimp in a volume of 10 $\mu$l per animal. All inoculations were performed with a 25-gauge needle (Terumo) mounted on an accurate syringe (P/N: 81001/00, 1710 LT, 100 $\mu$l, Hamilton Bonaduz). After inoculation, shrimp were placed in their individual 10l aquarium for 5 days. Dead animals were collected every 6h

**Sample analysis and counting of bacterial density.**

Dead and surviving shrimp after the end of experiment were washed once with 70% alcohol and twice with FASW. Afterwards, they were homogenized in FASW with a stomacher, serial diluted, and plated on MA with 100 mg/L rifampicin (MAR). The plates were incubated at 28°C for 24 h. For Vibrio enumeration of water samples, 10-fold serial dilutions of the samples were made in FASW, and then plated on MAR.

**Results (Table 3)**

The lethal titers in shrimp inoculated via intramuscular route were: $10^{4.16}$, $10^{4.37}$, $10^{4.16}$ $LD_{50}$/ml for the three experiments. Inoculation via peroral route did not result in death. Inoculaton via antennal gland was "successful" as it led to mortality in shrimp. Inoculation via the antennal gland resulted in lethal titers of $10^{2.50}$, $10^{2.50}$, $10^{2.32}$ $LD_{50}$/ml. This shows that bacteria can invade the shrimp's body when it is successful in entering the antennal gland. Compared with the intramuscular route, $10^{1.66}$, $10^{1.17}$, $10^{1.66}$ times more bacteria are necessary to kill a shrimp.

Table 3: Lethal titers of a *V. campbellii* stock in *P. vannamei* by different routes of inoculation.

**Experiment 1**

| Treatment | Dilution | Mortality rate % | Time of death (hpi) | | | | | Average time of dead shrimp (hpi) | Resulting lethal titer ($LD_{50}$/ml) |
| --- | --- | --- | --- | --- | --- | --- | --- | --- | --- |
| | | | Sh1 | Sh2 | Sh3 | Sh4 | Sh5 | | |
| Intramuscular injection | $10^{-2}$ | 100 | 6 | 6 | 6 | 6 | 6 | 6 | **$10^{4.16}$** |
| | $10^{-3}$ | 60 | 6 | 12 | 12 | - | - | 10 | |
| | $10^{-4}$ | | - | - | - | - | - | | |
| Per os inoculation | $10^{0}$ | | - | - | - | - | - | | |
| | $10^{-1}$ | | - | - | - | - | - | | |
| | $10^{-2}$ | | - | - | - | - | - | | |
| Antennal inoculation | $10^{0}$ | 60 | 12 | 12 | 18 | - | - | 14 | **$10^{2.5}$** |
| | $10^{-1}$ | 40 | 12 | 12 | - | - | - | 12 | |
| | $10^{-2}$ | | - | - | - | - | - | | |

**Experiment 2**

| Treatment | Dilution | Mortality rate | Time of death (hpi) | | | | | Average time of dead shrimp (hpi) | Resulting lethal titer ($LD_{50}$/ml) |
| --- | --- | --- | --- | --- | --- | --- | --- | --- | --- |
| | | | Sh1 | Sh2 | Sh3 | Sh4 | Sh5 | | |
| Intramuscular injection | $10^{-2}$ | 100 | 6 | 6 | 6 | 12 | 12 | 8.4 | **$10^{4.37}$** |
| | $10^{-3}$ | 80 | 6 | 6 | 12 | 12 | - | 9 | |
| | | | - | - | - | - | | | |
| Per os inoculation | $10^{0}$ | | - | - | - | - | - | | |
| | $10^{-1}$ | | - | - | - | - | - | | |
| | $10^{-2}$ | | - | - | - | - | - | | |
| Antennal inoculation | $10^{0}$ | 60 | 6 | 6 | 12 | - | - | 8 | **$10^{2.5}$** |
| | $10^{-1}$ | 40 | 12 | 12 | - | - | - | | |
| | $10^{-2}$ | | - | - | - | - | - | | |

**Experiment 3**

| Treatment | Dilution | Mortality rate | Time of death (hpi) | | | | | Average time of dead shrimp (hpi) | Resulting lethal titer ($LD_{50}$/ml) |
| --- | --- | --- | --- | --- | --- | --- | --- | --- | --- |
| | | | Sh1 | Sh2 | Sh3 | Sh4 | Sh5 | | |
| Intramuscular injection | $10^{-2}$ | 100 | 6 | 6 | 6 | 6 | 6 | 6 | **$10^{4.16}$** |
| | $10^{-3}$ | 60 | 6 | 6 | 12 | - | - | 8 | |
| | $10^{-4}$ | | | | | | | | |
| Per os inoculation | $10^{0}$ | | - | - | - | - | - | | |
| | $10^{-1}$ | | - | - | - | - | - | | |
| | $10^{-2}$ | | - | - | - | - | - | | |
| Antennal inoculation | $10^{0}$ | 60 | 6 | 6 | 12 | - | - | 8 | **$10^{2.32}$** |
| | $10^{-1}$ | 20 | 12 | - | - | - | - | 12 | |
| | $10^{-2}$ | | - | - | - | - | - | | |

From example 3 it is clear that Vibrio easily colonizes and kills the animals upon intra-antennal inoculation.

References

- Corteel, M. 2013. White spot syndrome virus infection in P. vannamei and M. rosenbergii:experimental studies on susceptibility to infection and disease. Ph D Dissertation, Ghent University, Belgium (ISBN 9789058643308)

- Escobedo-Bonilla, C., M. Wille et al. (2005). In vivo titration of white spot syndrome virus (WSSV) in specific pathogen-free Litopenaeus vannamei by intramuscular and oral routes. Diseases of aquatic organisms 66(2): 163.

- Felgenhauer, 1992 . Internal Anatomy of the Decapoda: An Overview. J V Microscopic Anatomy of Invertebrates Volume 10: Decapod Crustaceae, pages 45-75 © 1992 Wiley-Liss, Inc.

- Jiravanichpaisal, Bangyeekhun et al., 2001. Experimental infection of white spot syndrome virus in freshwater crayfish Pacifastacus leniusculus. Diseases of aquatic organisms 47(2): 151-157.

- Lin, S. H., C.H. Liou et al. 2000. The role of the antennal glands in ion and body volume regulation of cannulated Penaeus monodon reared in various salinity conditions. Journal of Aquatic Animal Health 10(3): 271-281.

- Reed L.J. and H. Muench, 1938. A simple method of estimating fifty per cent endpoints. American Journal of Epidemiology 27(3): 493-497.

**Claims**

1. A process to infect a crustacean with an infectious agent comprising:

   - collecting said crustacean,
   - collecting said infectious agent,
   - removing urine via the external pore of the antennal gland of said crustacean, and
   - inoculating an amount of said infectious agent into the external pore of the antennal gland of said crustacean.

2. A process according to claim 1 wherein said inoculation is undertaken with a catheter or plastic tube attached to a syringe containing said amount of infectious agent.

3. A process according to claim 2 wherein said syringe a 0.1 ml syringe attached to a 24G catheter.

4. A process according to any of claims 1-3, wherein said crustacean is a shrimp, prawn or crab.

5. A process according to claim 4 wherein said shrimp is Penaeus vannamei, Penaeus monodon, Penaeus indicus or Penaeus chinensis.

6. A process according to claim 5 wherein said prawn is a Macrobrachium species.

7. A process according to any of claims 1-6, wherein said infectious agent is a virus.

8. A process according to claim 7, wherein said virus is white spot syndrome virus.

9. A process according to any of claims 1-6, wherein said infectious agent is a bacterium.

10. A process according to claim 9, wherein said bacterium is a bacterium belonging to the genus Vibrio.

11. A process according to claim 10, wherein said bacterium is a bacterium causing early mortality syndrome.

12. A process to screen for crustaceans which are resistant to an infectious agent comprising infecting said crustaceans using a process according to any of claims 1-11 and determining resistance of said crustaceans to said infection.

13. A process to screen for compounds modulating resistance of crustaceans to an infectious agent comprising exposing said crustaceans to said compounds, infecting said crustaceans using a process according to any of claims 1-11 and determining resistance of said crustaceans to infection compared to resistance of crustaceans which are not exposed to said compounds.

**Patentansprüche**

1. Verfahren zum Infizieren eines Krebstiers mit einem Infektionserreger, umfassend:

   - Sammeln des Krebstiers,
   - Sammeln des Infektionserregers,
   - Entnehmen von Urin über die äußere Pore der Antennendrüse des Krebstiers und
   - Überimpfen einer Menge des Infektionserregers in die äußere Pore der Antennendrüse des Krebstiers.

2. Verfahren nach Anspruch 1, wobei das Überimpfen mit einem Katheter oder einem Kunststoffröhrchen, befestigt an einer Spritze, die die Menge an Infektionserreger enthält, durchgeführt wird.

3. Verfahren nach Anspruch 2, wobei die Spritze eine an einem 24G-Katheter befestigte 0,1 ml-Spritze ist.

4. Verfahren nach einem der Ansprüche 1-3, wobei es sich bei dem Krebstier um eine Garnele, eine Krabbe oder einen Krebs handelt.

5. Verfahren nach Anspruch 4, wobei es sich bei der Garnele um Penaeus vannamei, Penaeus monodon, Penaeus indicus oder Penaeus chinensis handelt.

6. Verfahren nach Anspruch 5, wobei die Krabbe eine Macrobrachium-Spezies ist.

7. Verfahren nach einem der Ansprüche 1-6, wobei es sich bei dem Infektionserreger um ein Virus handelt.

8. Verfahren nach Anspruch 7, wobei das Virus ein White-Spot-Syndrom-Virus ist.

9. Verfahren nach einem der Ansprüche 1-6, wobei es sich bei dem Infektionserreger um ein Bakterium handelt.

10. Verfahren nach Anspruch 9, wobei das Bakterium ein zur Gattung Vibrio gehörendes Bakterium ist.

11. Verfahren nach Anspruch 10, wobei das Bakterium ein EMS (early mortality syndrome) verursachendes Bakterium ist.

12. Verfahren zum Screening auf Krebstiere, die gegen einen Infektionserreger resistent sind, umfassend das Infizieren der Krebstiere unter Verwendung eines Verfahrens nach einem der Ansprüche 1-11 und Bestimmen der Resistenz der Krebstiere gegen die Infektion.

13. Verfahren zum Screening auf Verbindungen, die die Resistenz von Krebstieren gegen einen Infektionserreger modulieren, umfassend das Aussetzen der Krebstiere gegenüber diesen Verbindungen, das Infizieren der Krebstiere unter Verwendung eines Verfahrens nach einem der Ansprüche 1-11 und das Bestimmen der Resistenz der Krebstiere gegen eine Infektion im Vergleich zu der Resistenz von Krebstieren, die den Verbindungen nicht ausgesetzt wurden.

**Revendications**

1. Procédé d'infection d'un crustacé par un agent infectieux comprenant :

   - la collecte dudit crustacé,
   - la collecte dudit agent infectieux,
   - l'élimination d'urine via le pore externe de la glande antennaire dudit crustacé et
   - l'inoculation d'une quantité dudit agent infectieux dans le pore externe de la glande antennaire dudit crustacé.

**2.** Procédé selon la revendication 1, ladite inoculation étant mise en œuvre à l'aide d'un cathéter ou d'un tube en plastique attaché à une seringue contenant ladite quantité d'agent infectieux.

**3.** Procédé selon la revendication 2, ladite seringue étant une seringue de 0,1 ml attachée à un cathéter 24G.

**4.** Procédé selon l'une quelconque des revendications 1-3, ledit crustacé étant une crevette grise, une crevette bouquet ou un crabe.

**5.** Procédé selon la revendication 4, ladite crevette grise étant Penaeus vannamei, Penaeus monodon, Penaeus indicus ou Penaeus chinensis.

**6.** Procédé selon la revendication 5, ladite crevette bouquet étant une espèce de Macrobrachium.

**7.** Procédé selon l'une quelconque des revendications 1-6, ledit agent infectieux étant un virus.

**8.** Procédé selon la revendication 7, ledit virus étant un virus du syndrome des taches blanches.

**9.** Procédé selon l'une quelconque des revendications 1-6, ledit agent infectieux étant une bactérie.

**10.** Procédé selon la revendication 9, ladite bactérie étant une bactérie appartenant au genre Vibrio.

**11.** Procédé selon la revendication 10, ladite bactérie étant une bactérie causant un syndrome de mortalité précoce.

**12.** Procédé pour cribler les crustacés résistants à un agent infectieux comprenant l'infection desdits crustacés à l'aide d'un procédé selon les revendications 1 à 11 et la détermination de la résistance desdits crustacés à ladite infection.

**13.** Procédé pour cribler des composés modulant la résistance des crustacés à un agent infectieux comprenant la soumission desdits crustacés à l'action desdits composés, l'infection desdits crustacés à l'aide d'un procédé selon l'une quelconque des revendications 1-11 et la détermination de résistance desdits crustacés à une infection comparée à une résistance des crustacés qui ne sont pas soumis à l'action desdits composés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- experimental studies on susceptibility to infection and disease. **P. VANNAMEI ; M. ROSENBERGII.** Ph D Dissertation. Ghent University
- **ESCOBEDO-BONILLA, C. ; M. WILLE et al.** In vivo titration of white spot syndrome virus (WSSV) in specific pathogen-free Litopenaeus vannamei by intramuscular and oral routes. *Diseases of aquatic organisms,* 2005, vol. 66 (2), 163
- Internal Anatomy of the Decapoda: An Overview. **FELGENHAUER.** J V Microscopic Anatomy of Invertebrates Volume 10: Decapod Crustaceae. Wiley-Liss, Inc, 1992, vol. 10, 45-75
- **JIRAVANICHPAISAL ; BANGYEEKHUN et al.** Experimental infection of white spot syndrome virus in freshwater crayfish Pacifastacus Ieniusculus. *Diseases of aquatic organisms,* 2001, vol. 47 (2), 151-157
- **LIN, S. H. ; C.H. LIOU et al.** The role of the antennal glands in ion and body volume regulation of cannulated Penaeus monodon reared in various salinity conditions. *Journal of Aquatic Animal Health,* 2000, vol. 10 (3), 271-281
- **REED L.J. ; H. MUENCH.** A simple method of estimating fifty per cent endpoints. *American Journal of Epidemiology,* 1938, vol. 27 (3), 493-497